# EUROPEAN PATENT SPECIFICATION

(11) **EP 1 395 268 B1**
(45) Date of publication and mention of the grant of the patent: **19.01.2005**
(21) Application number: 02771628.1
(22) Date of filing: 23.05.2002
(51) Int. Cl.: A61K 33/24, A61L 27/04

(54) **A METHOD OF IMPLANTING HEAVY METAL SUCH AS NOBEL METAL, E.G. GOLD, AND METAL FOR USE IN IMPLANTATION**
VERFAHREN ZUR IMPLANTATION VON SCHWERMETALL, WIE Z.B. EIN EDELMETALL, Z.B. GOLD, UND METALL ZUR VERWENDUNG BEI DER IMPLANTATION
PROCEDE PERMETTANT D'IMPLANTER UN METAL LOURD, NOTAMMENT UN METAL NOBLE, PAR EXEMPLE, DE L'OR, ET METAL DESTINE A ETRE UTILISE DANS L'IMPLANTATION

(30) Priority: 25.05.2001 DK 200100840
(43) Date of publication of application: 10.03.2004
(73) Proprietor: Danscher, Gorm, 8000 Arhus C (DK)
(72) Inventor: Danscher, Gorm, 8000 Arhus C (DK)
(74) Representative: Larsen, Hans Ole
(86) International application number: PCT/DK2002/000344
(87) International publication number: WO 2002/094294

(56) References cited:
- WO-A-89/01764
- US-A- 4 606 354
- GORM DANSCHER: "In vivo liberation of gold ions from gold implants. Autometallographic tracing of gold in cells adjacent to metallic gold." HISTOCHEM CELL BIOL., vol. 117, 2002, pages 447-452, XP002902590

## Description

The invention relates to a heavy metal, such as noble metal e.g. gold for use in implantation in humans or animals.

The heavy metals such as noble metals gold and silver and mercury have been used since ancient times as cures for a wide range of diseases including tuberculosis, but only within the last 50 years, a more rational basis for the therapeutic use of gold and silver has emerged.
However, the use of metallic gold implants as a paramedical remedy for the treatment of rheumatism and pain is still considered irrational, because a scientific explanation of the postulated beneficial effects is lacking.
The authorized and scientifically well-founded use of gold in the medical world is mainly performed with various gold-thio compounds such as gold sodium thiomalate and recently Auronofin R, a drug suited for oral treatment of rheumatoid arthritis.

Already quite early on it was suggested that gold inhibits the lysosomal enzymes of phagocytotic cells in the inflamed synovial tissue.
Although knowledge of the effect of gold ions on the cellular immuno response is still sparse, it is recognized that gold ions are powerful inhibitors of macrophages and polymorphonuclear leucocytes, and the ability of the above-mentioned gold compounds to suppress inflammation in rheumatic joints was established a long time ago.

Gold ions are believed to inhibit antigen processing and to suppress NF-kappa B binding activity and I-kappa B-kinase activation, resulting in a reduced production of proinflammatory cytokines.
The use of gold implants originates from acupuncture where gold needles seem to have been used quite often.
In the early 1970s some US veterinarians started to treat dogs suffering from hip dysplasia with gold implants, and since then several veterinary surgeons and medical doctors have implemented the technique on their patients.
The possibility of silver-enhancing minuscule metallic gold clusters in tissues by autometallographic (AMG) silver enhancement was realized in 1981 and was soon implemented as a tool for enhancing colloidal gold particles bound to antibodies and enzymes.
Gold ions bound in gold complexes will give rise to nano sized clusters of metallic gold if subjected to reduction e.g. by radiating tissue with UV light.

It is realised that gold implants release gold ions, most likely by macrophage caused oxidation of the surface, which bind to species such as cyanide or sulfhydryl groups in tissue adjacent to the implant.
The gold-loaded molecules are then phagocytosed by cells close to the gold implants and finally accumulated in the lysosomes.

After reduction of the tissue sections, the resulting nanoclusters of gold atoms are visualized by AMG.

It is therefore hypothesized that the surface of the gold implants stimulates a reaction from the immune system, causing an oxidative liberation of gold ions that bind to the above-mentioned molecules.

The released gold ions, possibly AU(I), may exert their inhibitory effect on inflammation either directly by harming peptides or proteins by reacting chemically with them, or they may have to be converted to aurocyanide ions first. If in excess, Au(I) or Au(CN)⁻₂ might drift further into the intercellular space, bind to e.g. sulphydryl species and be phagocytosed by mast cells, macrophages and other cells farther away.
Based on the present knowledge it can be stated that the larger the surface of the gold implants is, the more gold ions are released and the farther away cell loaded with gold can be found.

Since it has thus been shown that it is gold ions released from pure gold that give the desired effect, it is accordingly an object of the invention to provide a heavy metal in which the greatest possible amount of metal ions may be released after implantation. It is likewise an object to apply miniscule gold particles, such as colloidal gold particles to internal and external surfaces, subjected to immunoreactive reactions with the purpose of damping the immunreaction.

The object of the invention is achieved by a heavy metal of the type defined in the introductory portion of claim 1, which is characterized in that the metal is formed by one or more pieces e.g. of gold, having a surface of a piece of metal having a given mass that is larger than the surface of a spherical or cylindrical piece of metal having the same mass.
Hereby, the desired and optimised levels of metal ions are obtained locally in human or animals.

When, as stated in claim 2, the metal is provided as colloidal gold particles, or the metal is mixed into a solution, or the metal is created as carrier substances to be dispensed as drops, or as air soles (sprays) or as ointments or crèmes or tablets or capsules, it is ensured that the release of gold particles can be adapted to certain treatments, such as internal or external treatment, eye treatments or the like.

Further advantages in treatments in connection with the examples mentioned in claim 2 are:
For treatment of respiratory immunoreactive maladies, it is an advantage if the colloidal gold containing solution is applied as a spray with a spray device, and for treatment of allergic reactions in the nose a nose spray can be used.
For downgrading immunoresponses in the gastrointestinal canal, including the mouth, colloidal gold particles are mixed into the content of tablets mixtures.
Finally, for reducing the intensity of allergic skin inflammations and eczemas the colloidal gold particles can be dispensed in crèmes, ointments or the like.

A practical provision of gold with a large surface may be achieved as stated in claim 3 in that the threads of gold are wound as spring like coils with one or more windings.

Likewise, a large surface of the gold may be ensured in that, as stated in claim 4 that the gold implants are created by reticulated or foiled gold or by foamed gold and as stated in claim 5, that the gold implants are created of sintered metal particles e.g. gold particles.

The invention will be illustrated more fully below with reference to the drawing, in which
Fig 1, pictures A - I show micrographs of autometallographically (AMG) developed tissue sections, from tissue samples taken near a gold implant, while
Fig 2, pictures A - D show electron micrographs from gold-implanted rat brain.

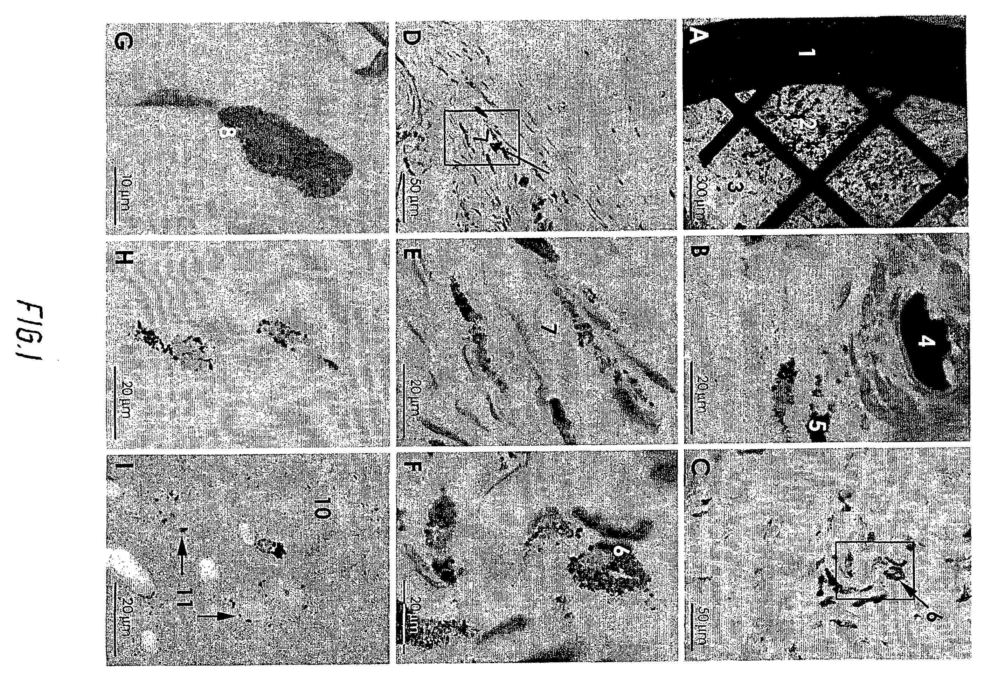

In fig. 1, pictures A - H show a 5-µm methacrylate embedded connective tissue section.

In fig 1, picture I shows an Erpon embedded 3-µm thick brain section.
All sections were counterstained with toluidine blue.

In picture A, it will be seen that part of a gold grid 1 dominates the picture. In the closed mesh windows cells are loaded with silver-enhanced gold clusters 2.
In the open window far less cells are stained, see numeral 3.

In picture B, part of a gold grid mesh is seen at 4 in the upper part of the picture. The dusty appearance of the tissue close to the gold implant represents gold clusters located outside cells. The two loaded cells, cf. 5 further away, are believed to be macrophages.

Picture C shows a rather intense AMG staining of macrophages, mast cells and fibroblasts in the connective tissue from subcutis.
The gold containing cells, cf. the numeral 6, was located only a few mm from a gold implant.
Picture D was taken from an AMG stained section of a demineralised piece of the skull bone. The gold implant was placed in a drilled hole in the skull where it became gradually embedded in the bone.
Newly created bone lamella, cf. numeral 7, encircled the connective tissue implant connective tissue.
Macrophages and fibroblasts loaded with AMG grains are visible in the connective tissue.

Picture E shows a greater magnification of the square than in fig. D, and picture F a greater magnification of the square in C.

Picture G shows a photomicrograph of mast cells from gold implanted subcutis.
It is noted that only part of the mast cell granules contained gold that is indicated by numeral 8.

Picture H shows a typical fibroblast from connective tissue surrounding a gold implant for six weeks.

Picture I is taken from a rat neocortex. The implant was located approximately 1 mm , cf. numeral 10, from the AMG stained astrocyte and neurons.

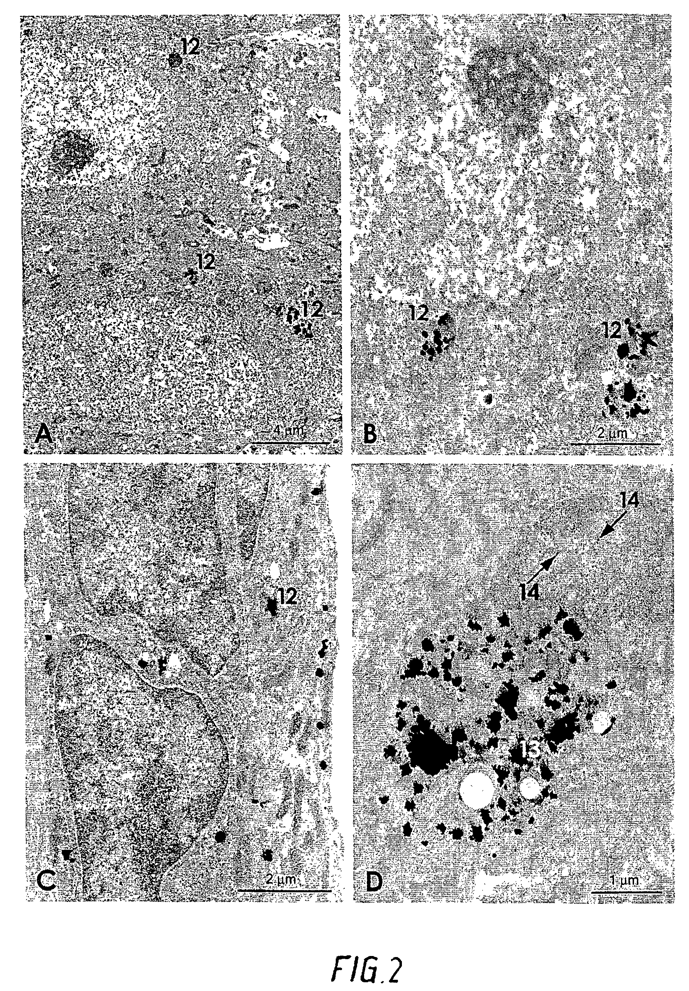

Fig. 2, picture A shows two minor intemeurons from neocortex.
Note the exclusive localization of gold in lysome-like organelles, cf. numeral 12 in fig 2, picture A to C.
The gold grid based implant was located approximately 1.5 mm from the stained neurons.

Picture B shows gold-loaded neuron from caudate putamen.

Picture C shows astrocytes adjacent to a capillary (c).

Picture D shows astrocytes loaded with gold. The AMG grains seem to be free in the cytoplasm, and the nucleus reveals tiny AMG particles in the euchromatine, cf. the arrows having numeral 14.

In the following an experiment carried out by the inventor will be explained:

Twenty-seven adult rats had threads of pure gold implanted in the body. The compact threads were 1.25 mm long and 0.5 mm thick, and weighed approximately 5.5 mg. They were cut from a twenty-four carat gold thread. The gold threads were implanted close to arthritic joints in an attempt to decrease pain and inflammation.
Another ten rats had implanted coiled-up gold grids with a diameter of 3.0 mm and weighing 0.5 mg. The animals were anaesthetized with Nembutal, and the gold threads were implanted by a needle and a stiletto into different parts of the body including the brain close to joints.
At different intervals of survival (10 days to 3 months) the animals were re-anaesthetized and transcardially perfused with 3% glutaraldehyde in a 0.1 M phosphate buffer.
The tissues holding the gold threads or grids were excised and placed in the fixative for at least two hours.
The tissue blocks were then either frozen on a stage and cut into 20-µm sections in a cryostat, or they were cut into 100- µm sections in a vibratome.
Every second glass slide was radiated for 30 min with UV light (wavelength 365 nm) upon which all specimens were AMG developed floating in the AMG developer. From the developed vibratome sections, areas of interest were cut out with razor blade and treated with 0.5 % osmium tetraoxide for 30 minutes, rinsed, dehydrated and embedded in Epon. Some of the tissue blocks were not fixed in osmium, but directly embedded in methacrylate.

Every second glass slide with cryostat or methacrylate sections was radiated with UV light for 30 min. and placed in a jar, covered by the AMG developer and placed in a 26 °C water bath.
A light-tight hood covered the set-up through the 60 minutes of development. The AMG developer was then replaced by a 5% thiosulphate solution for 10 min in order to remove all silver ions from the sections, and ultimately the sections were rinsed several times in distilled water and counterstained with toluidine blue (15).
Semi-thin sections were cut from the Epon blocks and placed on glass slides. They were counterstained with toluidine blue (1%) and analyzed in the light microscope. When photos had been taken, selected sections were reembedded with a drop of Epon on top of a blank Epon. Ultra-thin sections were cut, stained with uranyl and lead, and analysed in an electron microscope.

Controls included sections from tissues not adjacent to gold implants and sections treated with a 10% potassium cyanide solution for 30 min. Blocks of the tissue surrounding the implants were analyzed with Proton Induced X-ray Emission spectroscopy (PIXE). The 20-µm thick cryostat sections were placed on framed micropore polystyrene membranes so thin that the energy loss due to small angle scattering was negligible.

This experiment shows that connective tissue blocks from skin and joints contained a rim of gold ion-imbibed tissue around the gold implants, cf. also fig. 1, pictures A and B. In this juxta-positioned implant tissue, macrophages and mast cell were the first to show accumulation of gold, cf. fig. 1, pictures B - H. already after 14 days the first traces of AMG enhanced gold clusters could be observed, and after one to two months an increasing amount of loaded cells including fibroblasts were seen. Not all macrophages or mast cells showed an uptake of gold, quite the reverse. Even close to the gold implants some macrophages and mast cells completely void of gold were observed. The most heavily loaded cells, whether macrophages or mast cells, showed signs of degeneration. In cells showing degeneration, EM magnifications showed gold accumulations that were not enclosed by a membrane, but seemed to be located free in the cytoplasm. The pattern gave the impression that lysosomes/vesicles take up increasing amounts of gold until the enclosing membrane bursts. Cells with free gold accumulations sometimes had gold accumulations in the nucleus. Cells closer to the gold implants were more loaded than cells farther away, and macrophages and mast cells stained more heavily than fibroblasts. Fibroblasts became loaded only after long periods of time, approximately two months in the case of rod-shaped gold threads, but faster if coiled-up gold grids were used as implants.
Also the longer the implant had been in the tissue, the more stained cells and the broader the rim of gold-imbibed tissue.
The coiled-up grid implants, having a substantially increased surface compared to the rod-shaped gold threads, released far more gold ions, recorded as a drastic increase in the amount of stained cells and in the staining intensity in the individual cells. Kidneys and livers from implanted animals were examined in order to test whether gold ions spread systemically.
In cases where more than one coiled-up grid was implanted, a faint staining in the proximal tubule of the kidney was recorded in some animals, while the liver was void of AMG grains.
If coiled-up grids were implanted directly in the kidney, some release of gold ions could be detected in nearby cells, but of all the implanted tissues those in the kidney caused the least release of gold ions.
The compact gold threads implanted in the brain revealed a tiny rim of imbibed cells closely associated to the implant, but gold ion-loaded neurons and glia cells could be traced further from the rim, cf. fig. 1, picture I. The coiled-up grid implants resulted in an up to several millimetre wide zone of gold-loaded neurons and glia cells around the implant. Again some neurons and glia cells were completely void of gold. In a few animals the implant had been embedded in connective tissue, and no AMG grains were detected in the surrounding neurons and glia cells.
In both glia cells and neurons gold was found to be located in lysosome-like vesicles, cf. fig. 2, pictures A - D. The loaded vesicles were spread throughout the neuronal somata and into the major dendrites. Most of the gold-containing glia cells seemed to be protoplasmatic astrocytes, cf. fig. 2, pictures C and D, but the possibility that oligodendroglia and microglia take up gold cannot be excluded at this early stage.
In addition to the fact that AMG staining was observed only in tissues relatively close to the gold implants, several controls were introduced in order to ensure that the staining was caused by gold clusters: the tissue sections were treated with 10% aqueous potassium cyanide for 30 minutes. This treatment resulted in a complete removal of the catalyst, and the following AMG development resulted in blank sections. The multi-element analysis of cryostat sections with PIXE showed that the tissue surrounding gold implants contained 38 p.p.m. gold, while tissue taken 1 cm from gold implant was void of gold (<0.39 p.p.m.).

Even that the invention has been explained in connection with certain embodiments, a few remarks concerning possible further embodiments are described below:
1) The possibility of surface treatment of metal implants e.g. gold and colloidal gold particles with molecules that enhance/decrease the amount of released metal ions over time.
2) Adhering molecules to such surfaces that support the therapeutic effects of metal ion release.
3) Use of implants or colloidal metallic surfaces as carrier of therapeutic molecules and ions .e.g. molecules that is slowly released from such surfaces placed on epithelial surfaces or in tissues.
4) Use of metallic molecules that ensure that the colloidal metal particles are taken up by certain types of cells.
5) Use of molecules that ensure that the colloidal gold is concentrated in a particular cell organelle.
6) Use of alloys in the preparation of an implant for implantation in humans or animals.

## Claims

1. A heavy metal, such as noble metal e.g. gold for use in implantation in humans or animals, **characterized in that** the metal is formed by one or more pieces e.g. of gold, having a surface of a piece of metal having a given mass that is larger than the surface of a spherical or cylindrical piece of metal having the same mass.

2. A heavy metal according to claim 1, **characterized in that** the metal is provided as colloidal gold particles, or the metal is mixed into a solution, or the metal is created as carrier substances to be dispensed as drops, or as air soles (sprays) or as ointments or crèmes or tablets or capsules.

3. A heavy metal according to claims 1 - 2, **characterized in that** the threads of gold are wound as spring like coils with one or more windings.

4. A heavy metal according to claims 1 - 3, **characterized in that** the gold implants are created by reticulated or foiled gold or by foamed gold.

5. A heavy metal according to claims 1 - 4, **characterized in that** the gold implants are created of sintered metal particles, e.g. gold particles.

## Patentansprüche

1. Schwermetall, wie ein Edelmetall, z.B. Gold, für die Verwendung in Implantationen in Menschen oder Tieren, **dadurch gekennzeichnet, dass** das Metall aus einem oder mehreren Teilen, z.B. aus Gold, gebildet wird und eine Oberfläche aus einem Metallstück mit einer gegebenen Masse aufweist, die größer ist als die Oberfläche eines kugelförmigen oder zylindrischen Metallstückes mit derselben Masse.

2. Schwermetall gemäß Anspruch 1, **dadurch gekennzeichnet, dass** das Metall als kolloidale Goldteilchen zur Verfügung gestellt wird, oder dass das Metall in eine Lösung gemischt wird, oder dass das Metall als Trägersubstanzen hergestellt wird, die als Tropfen oder als Luftsole (Sprays) oder als Salben oder Cremes oder Tabletten oder Kapseln abgegeben werden.

3. Schwermetall gemäß den Ansprüchen 1 bis 2, **dadurch gekennzeichnet, dass** die Goldfäden wie federähnliche Spiralen mit einer oder mehreren Windungen aufgewickelt werden.

4. Schwermetall gemäß den Ansprüchen 1 bis 3, **dadurch gekennzeichnet, dass** die Goldimplantate mittels retikulärem Gold oder mittels Goldfolie oder mittels geschäumtem Gold geschaffen werden.

5. Schwermetall gemäß den Ansprüchen 1 bis 4, **dadurch gekennzeichnet, dass** die Goldimplantate aus gesinterten Metallteilchen, z.B. Goldteilchen, geschaffen werden.

## Revendications

1. Métal lourd, tel qu'un métal noble, par exemple de l'or, pour utilisation dans une implantation dans des humains ou des animaux, **caractérisé en ce que** le métal est formé par un ou plusieurs morceaux par exemple d'or, comportant une surface d'un morceau de métal ayant une masse donnée qui est plus grande que la surface d'un morceau sphérique ou cylindrique d'un métal ayant la même masse.

2. Métal lourd selon la revendication 1, **caractérisé en ce que** le métal est fourni en tant que particules d'or colloïdal, ou le métal est mélangé dans une solution, ou le métal est créé en tant que substances de support pour être administré en gouttes, ou en aérosols (vaporisateurs) ou en pommades ou crèmes ou comprimés ou capsules.

3. Métal lourd selon les revendications 1 à 2, **caractérisé en ce que** les fils d'or sont enroulés en ressort comme des bobines avec un ou plusieurs enroulements.

4. Métal lourd selon les revendications 1 à 3, **caractérisé en ce que** les implants en or sont créés par de l'or réticulé ou en feuille ou par de l'or expansé.

5. Métal lourd selon les revendications 1 à 4, **caractérisé en ce que** les implants en or sont créés avec des particules métalliques frittées, par exemple des particules d'or.
